# EUROPÄISCHE PATENTSCHRIFT

(11) **EP 1 212 976 B1**
(45) Veröffentlichungstag und Bekanntmachung des Hinweises auf die Patenterteilung: **10.03.2004**
(21) Anmeldenummer: 01128940.2
(22) Anmeldetag: 05.12.2001
(51) Int. Cl.: A61B 1/06

(54) **Endoskop**
Endoscope
Endoscope

(30) Priorität: 07.12.2000 DE 10061107
(43) Veröffentlichungstag der Anmeldung: 12.06.2002
(73) Patentinhaber: Henzler, Marc, 78532 Tuttlingen (DE)
(72) Erfinder: Henzler, Marc, 78532 Tuttlingen (DE)
(74) Vertreter: Patentanwälte Westphal, Mussgnug & Partner

(56) Entgegenhaltungen:
- WO-A-97/11634
- DE-U- 29 613 103
- DE-U- 29 805 624

## Beschreibung

Die Erfindung betrifft ein Endoskop mit den im Oberbegriff des Patentanspruchs 1 angegebenen Merkmalen.

Mit einem derartigen Endoskop können z.B. therapeutische oder diagnostische Eingriffe nach minimal invasiven Verfahren durchgeführt werden. Auch kann dieses Endoskop z.B in der technischen Endoskopie als sogenanntes Technoskop eingesetzt werden.

Diese Endoskope weisen einen in den Hohlraum einzuführenden Schaft auf, an dessen proximalen Ende das Bild mittels einer speziellen Optik, vorzugsweise eines Stablinsensystems, auf das in einem Okular befindlichen Objektiv projiziert wird. Das derart erzeugte Bild der Operationsfläche oder des einzusehenden Hohlraums wird mittels einer Kamera oder eines über Signalleitung und Steuereinheit (Kameracontroler) verbundenen Monitors dargestellt.

Üblicherweise wird das Beleuchtungslicht über einen Lichtleiter, bestehend aus Glasfaserbündeln oder einem Flüssigleiter, von einer externen Lichtquelle an das Endoskop geleitet und dort über eine Kupplung mit weiteren Lichtleitfasern im Inneren des Endoskops verbunden, welche das Licht dem distalen Ende des Endoskops zuführen.

Dieses herkömmliche Beleuchtungssystem ist technisch aufwendig. Die üblicherweise verwendeten Kaltlichtquellen, z.B. Xenon- oder Halogenlampen, erfordern eine relativ hohe elektrische Leistung und sind mit technisch aufwendigen Maßnahmen zu beschalten und zu kühlen, um auch bei langen Lichtleitern und hohen Einkoppel- bzw. Kupplungsverlusten genügend Licht an das distale Ende des Endoskops zu bringen. Auch haben die o.g. Lichtquellen keinen optimalen Wirkungsgrad.

Hinzu kommt eine hohe Ausfallrate wegen der kurzen Lebensdauer dieser Lichtquellen und der mechanisch stark beanspruchten Lichtleitkabel, was zu hohen Instandhaltungskosten und damit zur Herabsetzung der Wirtschaftlichkeit führt.

Aus DE 296 13 103 U1, DE 298 12 048 U1 sind Endoskope bekannt, bei welchen zur Beleuchtung Licht emittierende Dioden, sogenannte LED's, am distalen Ende des Endoskops angebracht sind.

Vorteil dieser am distalen Ende angebrachten Beleuchtung ist, dass Einkopplungsverluste im Lichtleiter und Übertragungsverluste durch lange Lichtleiter vermieden werden. Ferner kann am distalen Ende des Endoskops ein CCD-Chip (Charge Coupled Devises-Chip) als Bildaufnehmer vorgesehen sein, wodurch Helligkeitsverluste auf Grund der Luft / Glasübergänge vermieden werden.

Mit DE 296 13 103 U1 wird die Verwendung von LED-Chips für die Darstellung der Farben Rot, Blau und Grün zur Ausnutzung der Fluoreszenzanregung der im karzinogenen Gewebe angesammelten Fluoreszenzstoffe vorgeschlagen. Diese Anwendung macht die Verwendung eines Chips mit CCD-Elementen für Schwarz-Weiß Übertragung erforderlich, welchen dichroiche Filter mit einer Durchlässigkeit von > 56 nm vorgeschaltet werden müssen. Diese Anordnung, insbesondere die Verwendung der genannten Schwarz-Weiß-CCDs, schränkt die Anwendungsgebiete des Endoskops jedoch erheblich ein.

In DE 299 10 795 U1 ist ein Endoskop beschrieben, bei welchem mehrere Beleuchtungseinheiten axial hintereinander liegend im Schaft angeordnet sind, deren Licht über Lichtleiter und den am proximalen Ende befindlichen ARRAYs von LED's zugeführt wird. Diese Anordnung erfordert durch die erhöhte Lichtleistung der im Gesamtsystem verwendeten größeren Anzahl von Beleuchtungseinheiten und der Verluste durch die Einkopplungen der einzelnen Beleuchtungseinheiten in Lichtleitern einen erhöhten Energiebedarf. Ausserdem ist die Einkopplung der Lichtleiter und deren Unterbringung im Schaft aufwendig und damit sehr kostspielig.

Der vorliegenden Erfindung liegt die Aufgabe zugrunde, ein Endoskop zu schaffen, bei welchem die Handhabung und Ergonometrie verbessert sind und dessen Fertigungskosten durch eine Verbesserung der Integration des Beleuchtungssystems verringert sind. Außerdem soll der Außendurchmesser des Endoskopschaftes möglichst klein sein.

Diese Aufgabe wird gemäß der Erfindung mit den Merkmalen des Anspruchs 1 gelöst.

Nach der Grundidee der Erfindung sind als Beleuchtungseinheiten LED's auf ringförmigen, lichtdurchlässigen Trägern, nachstehend auch Leuchtringe genannt, vorgesehen, welche zwischen innerem und äußerem Hüllrohr, also die Optik umgebend, angeordnet sind, wobei mehrere Träger zwischen proximalem und distalem Ende des Schaftes im Ringraum zwischen innerem und äußerem Hüllrohr axial, kaskadenartig hintereinander angeordnet sind.

Mit einer derartigen Anordnung wird eine wesentlich größere Leuchtdichte als bei bekannten Endoskopen dieser Art erzielt. Glasfaserleiter im Inneren des Schaftrohres entfallen ganz.

Hierdurch wird die Fertigung vereinfacht und verbilligt, wobei sehr geringe Schaftdurchmesser realisierbar sind.

Wie mit Anspruch 2 vorgeschlagen, können die die Beleuchtungseinheiten bildenden LED's auf die als ringförmige Glasplättchen ausgebildeten Träger aufgebondet , also maschinell verdrahtet, werden und die Leiterbahnen zur Stromversorgung der LED's durch Bedampfen, vorzugsweise Goldbedampfung, hergestellt werden. Hierdurch wird eine außerordentlich hohe Fertigungsdichte erreicht, welche mit herkömmlich vorgefertigten LED's oder mit SMD LED's (Surface Mounted Devices-LED's) nicht zu erreichen ist.

Eine weitere Erhöhung der Leuchtdichte läßt sich dadurch erzielen, dass gemäß Anspruch 3 hinter dem proximal letzten Träger eine das Licht reflektierende Verspiegelung angeordnet ist, die gemäß Anspruch 4 aus einer aufgedampften Metallschicht bestehen kann. Dem gleichen Zweck dient der Vorschlag nach Anspruch 5, die einander zugewandten Flächen des inneren und äußeren Hüllrohrs, zwischen welchem die Träger mit den LED's angeordnet sind, ganz oder teilweise mit einer reflektierenden Schicht auszustatten.

Gemäß Anspruch 6 ist in an sich bekannter Weise das distale Ende des Schaftes mit einer lichtdurchlässigen Platte oder auch einer Linse abgeschlossen.

Weitere Maßnahmen zur wirtschaftlichen Fertigung der Träger für die Lichteinheiten sind Gegenstand der Ansprüche 7 bis 10.

Die Stromversorgung der LED's kann gemäß Anspruch 11 über elektrische Zuleitungen erfolgen, welche im Ringraum zwischen innerem und äußerem Hüllrohr verlaufen und mit einem Steckkontakt elektrisch verbunden sind. In diesen Steckkontakt ist ein der Stromzuleitung dienender Stecker einsteckbar. Hierdurch ist das Endoskop in sehr einfacher Weise an- und abzukuppeln.

Nach einem weiteren Vorschlag gemäß Anspruch 12 ist eine berührungslose Stromversorgung der LED's möglich, wenn die elektrischen Zuleitungen mit einer im Okular angeordneten Induktionsspule verbunden sind. In dieser Spule kann mittels einer diese berührungslos umgebenden, ein magnetisches Feld erzeugende Induktionsspule ein Strom erzeugt werden, welcher den LED's zugeführt wird.

Durch diese Maßnahme werden Handhabung und vor allem auch die Reinigungsmöglichkeiten, insbesondere die Autoklavierbarkeit, weiter verbessert.

Alternativ können gem. Anspruch 13 bei einer Parallel-Beschaltung der LED's auch die Hüllrohre als Leiter verwendet werden. Bei einer Verlötung der Leuchtringe mit den Hüllrohren kann über diese vorteilhafterweise Wärme abgeleitet werden.

Durch den Verzicht auf externe Lichtquellen, sowie ein diese mit dem Endoskop verbindendes Lichtleitkabel, werden Herstellungs- und Unterhaltskosten eines derartigen Systems minimiert. Beschaffungskosten für Lichtleitkabel und Gasentladungslampen entfallen, die Reparaturanfälligkeit ist reduziert, was insgesamt zu einer höheren Zuverlässigkeit des Systems führt.

Neben weiß scheinenden LED's können monochromatisch leuchtende LED's verwendet werden, wie mit Anspruch 14 vorgeschlagen ist. In diesem Fall kann durch Wellenkonvektion mittels des von den LED abgegebenen roten, grünen und blauen Lichtes weißes Licht erzeugt werden, indem die Komplementärfarben in einem bestimmten Verhältniss zugemischt werden.

Maßnahmen zur Bildwiedergabe sind mit den Ansprüchen 15 und 16 angegeben.

Der Gegenstand der Erfindung ist nachstehend anhand in der Zeichnung dargestellter Ausführungsbeispiele näher erläutert. In der Zeichnung zeigen
- Figur 1: Längsschnitt eines Endoskopes nach einem ersten Ausführungsbeispiel gem. der Erfindung in schematischer Darstellung, in dessen Schaftrohr Leuchtringe angeordnet sind,
- Figur 2: Längsschnitt eines Schaftrohres nach einem zweiten Ausführungsbeispiel in schematischer Darstellung, in welchem Leuchtringe und als optoelektrische Bilderzeugungseinrichtung ein CCD angeordnet sind,
- Figur 3: perspektivische Darstellung eines einzelnen Leuchtringes gem. der Erfindung und
- Figur 4: schematisches Schaltbild einer Anordnung mit erfindungsgemäßem Endoskop und Betrachtungseinrichtung

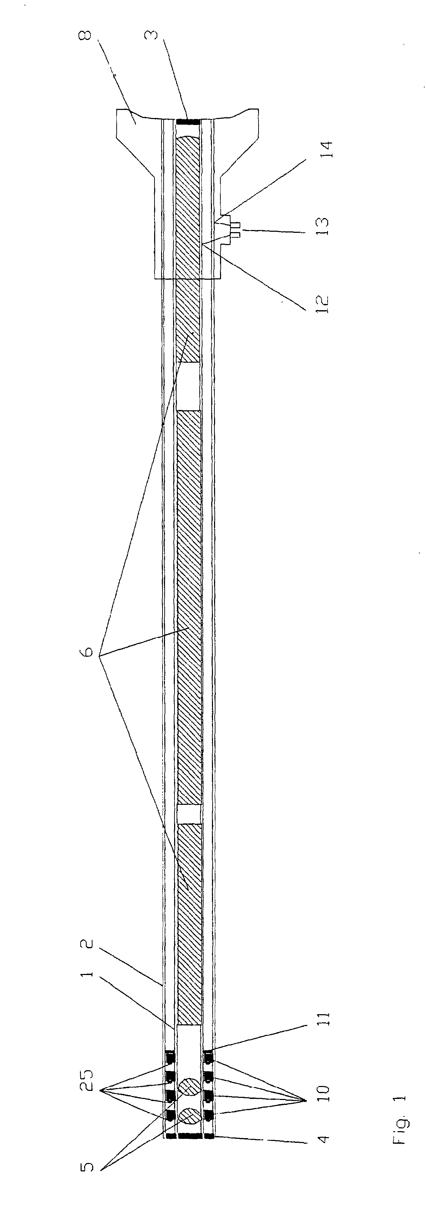

Figur 1 zeigt in schematischer Darstellung ein erfindungsgemäß ausgestaltetes Endoskop nach einem ersten Ausführungsbeispiel.

Dieses Endoskop besteht im wesentlichen aus einem Schaft mit innerem Hüllrohr 1 und äußerem Hüllrohr 2, an dessen proximalem Ende ein Okularteller 8 vorgesehen ist und dessen distales Ende mit einer Abschlussplatte 4 aus Glas flüssigkeitsdicht verschlossen ist. Im inneren Hüllrohr 1 ist ein aus zwei Linsen bestehendes Linsensystem 5 angeordnet. Das von diesem erzeugte Bild wird über das nachfolgend im inneren Hüllrohr 1 befindliche Stablinsensystem 6 auf die im Okularteller 8 befindliche, aus Glas bestehende Abschlussplatte 3 projiziert, wo es vom Benutzer betrachtet oder mittels einer Kamera aufgenommen werden kann.

Am distalen Ende zwischen innerem und äußerem Hüllrohr 1 und 2 sind axial hintereinander Leuchtringe, nämlich transparente Trägerringe 10 mit auf diesen aufgebondeten Leuchtdioden 25 axial hintereinander angeordnet. Die Rückseite des bezüglich des Okulars 8 vorderen Leuchtringes ist mit einer Verspiegelung 11 versehen. Bei dieser Anordnung gelangt das von den Leuchtdioden 25 aller Leuchtringe erzeugte Licht zum distalen Ende, wo es durch die distale Abschlussplatte 4 austreten kann. Zu einer noch besseren Ausnutzung des Lichtes können die einander zugewandten Innenflächen des inneren und äußeren Hüllrohres 1 und 2 im Bereich der Leuchtringe 10 verspiegelt sein.

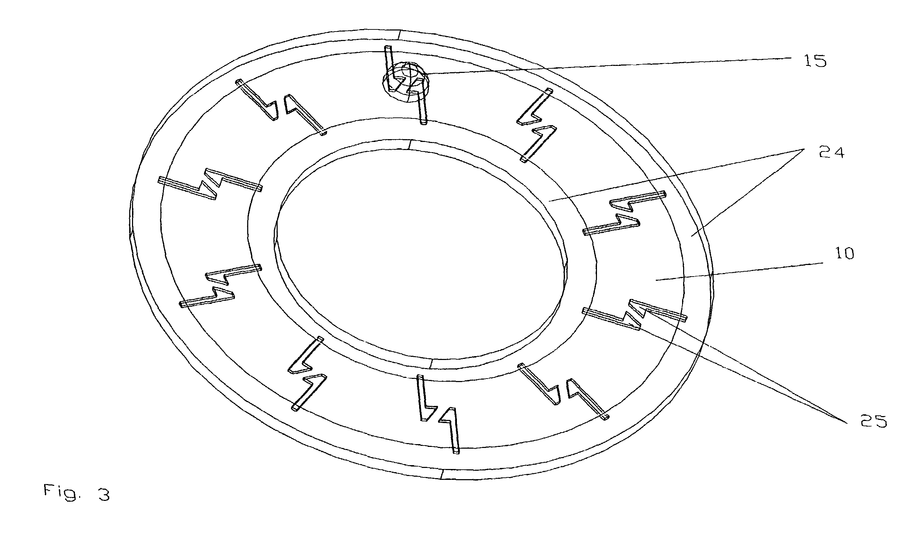

Die auf den ringförmigen Trägern 10 angeordneten Leuchtdioden 25 sind vorzugsweise parallel geschaltet, wie der Darstellung gemäß Fig. 3 entnommen werden kann. Ihre elektrischen Anschlüsse sind als Padbeschichtung 24 ausgebildet. Somit kann die Stromversorgung der LED's 25 über die Hüllrohre 1 und 2 erfolgen, welchen über Verbindungsleiter 12 bzw. 14 mit einem am Okularteller 8 vorgesehenen elektrischen Steckkontakt 13 verbunden sind.

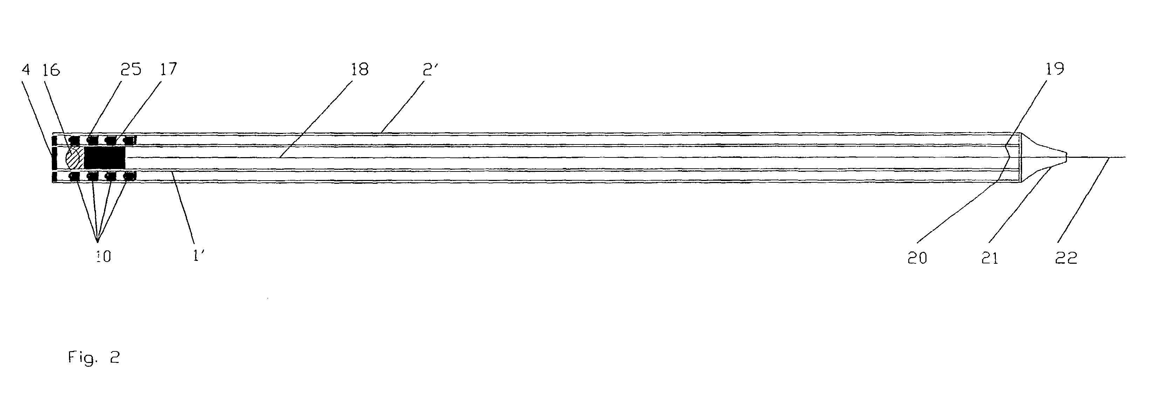

Beim zweiten Ausführungsbeispiel gem. Figur 2 ist am distalen Ende des Schaftrohres, nämlich innerhalb des inneren Schaftrohres 1', eine opto-elektronische Bilderzeugungseinheit 17 vorgesehen, welcher distalseitig die Sammellinse 16 zugeordnet ist. Im übrigen sind auch bei diesem Ausführungsbeispiel am distalen Ende die oben beschriebenen Leuchtringe 10 vorgesehen. Die von der opto-elektronischen Bildeinheit 17 erzeugten Bildsignale werden über die zentrale Signalleitung 18, welche bei der Abschlusskappe 21 endet, weitergeleitet und über das externe Anschlusskabel 22 dem nicht dargestellten Monitor zugeführt.

Die Stromversorgung der auf den Leuchtringen 10 vorgesehenen Leuchtdioden 25 erfolgt in gleicher Weise wie bei dem Ausführungsbeispiel gem. Fig. 1 über inneres und äußeres Hüllrohr 1' und 2'. Über Leiter 19 und 20 sind diese Hüllrohre am proximalen Ende mit dem externen Anschlusskabel 22 verbunden.

Mit Fig. 3 ist perspektivisch ein enzelner Leuchtring dargestellt. Der Leuchtring weist einen aus Glas bestehenden Träger 10 auf, auf welchem eine Mehrzahl von Leuchtdioden, die aus Kathode und Anode 25 gebildet sind, angeordnet sind. Die Kathoden und Anoden 25 dieser Leuchtdioden sind mit einer transparenten Verklebung, einem sogenannten Bubble, abgedeckt. Als Bubblematerial eignet sich Epoxydharz, Urethanakrylat oder Silikon. Diese Materialien können mit einem Fluoreszenzstoff versetzt werden, wodurch das von den LED erzeugte blaue Licht in weißes Licht umgewandelt wird, was unter dem Namen Lumineszenz-Wellenlängenkonversion bekannt ist. Mit diesen UBH-LED (Ultra-High-Bright) als Lichtquelle ist ein emittierter Strahlungsbereich zu erreichen, welcher vollständig das Spektrum von weißem Licht, das für das menschliche Auge sehbar ist, darstellt.

Die Stromversorgung der Dioden erfolgt über ringförmige Pads 24, welche im eingebauten Zustande mit den inneren und äußeren Hüllrohren 1 und 2 elektrisch verbunden, vorzugsweise verlötet, sind.

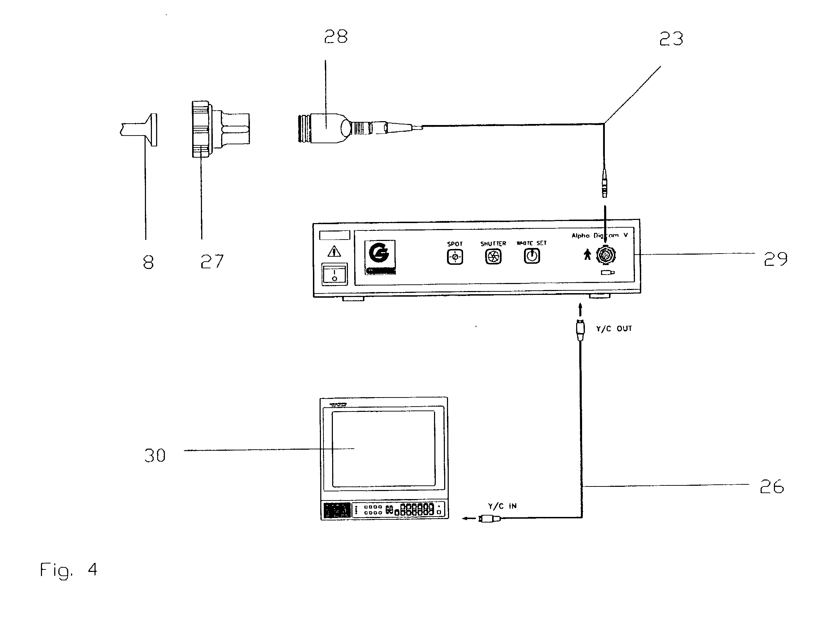

Das schematische Schaltbild gem. Fig. 4 zeigt eine mögliche Beschaltung des erfindungsgemäßen Endoskops, von welchem hier lediglich der Okularteller 8 gezeigt ist. Auf diesen Okularteller 8 ist ein Objektiv 27 aufklemmbar. An das Objektiv 27 lässt sich der Kamerakopf 28 ankoppeln. Die vom Kamerakopf 28 aufgenommenen Signale werden über das Verbindungskabel 23 der Steuereinheit 29 zugeführt, wo sie zur Erzeugung der Monitorsignale verarbeitet werden. So kann das vom Endoskop erzeugte Bild mit einem Monitor 30, welcher über das Kabel 26 an die Steuereinheit 29 angeschlossen ist, betrachtet werden.

### Bezugszeichenliste

- 1: inneres Hüllrohr des Schaftes
- 2: äußeres Hüllrohr des Schaftes
- 3: proximale Abschlussplatte aus Glas im Okularteller 8
- 4: distale Abchlussplatte
- 5: Linsensystem
- 6: Stablinsensystem
- 7: --
- 8: Okularteller
- 9: - -
- 10: ringförmiger Träger
- 11: Verspiegelung
- 12: Verbindungsleiter zwischen innerem Hüllrohr 1 und Steckkontakt 13
- 13: Steckkontakt
- 14: Verbindungsleiter zwischen äußerem Hüllrohr 2 und Steckkontakt 13
- 15: Bubble
- 16: Sammellinse
- 17: opto-elektrische Bilderzeugungseinheit
- 18: Signalleitung
- 19: Verbindungsleiter zwischen innerem Hüllrohr 1' und Anschlusskabel 22
- 20: Verbindungsleiter zwischen äußerem Hüllrohr 2' und Anschlusskabel 22
- 21: Abschlusskappe
- 22: Anschlusskabel
- 23: Verbindungskabel zwischen Kamerakopf 28 und Steuereinheit 29
- 24: Padbeschichtung als Kontaktierungsring
- 25: lichtemittierende Diode (LED), bestehend aus Kathode und Anode
- 26: Verbindungskabel zwischen Steuereinheit 29 und Monitor 30
- 27: Objektiv mit Klemmvorrichtung für den Okularteller 8
- 28: Kamerakopf
- 29: Steuereinheit
- 30: Monitor

## Patentansprüche

1. Endoskop mit einem Schaft mit koaxialen äußeren und inneren Hüllrohren (1, 2), einer im inneren Hüllrohr (2) angeordneten Optik, vorzugsweise einer Stablinsenoptik (6), und einem am distalen Ende vorgesehenen Linsensystem (5) bzw. einer Sammellinse (16) sowie im Schaft angeordneten Beleuchtungseinheiten (10) mit das innere Hüllrohr (2) kranzförmig umgebenden , lichtemittierenden Dioden (25) (LED's) zur Erzeugung des Beleuchtungslichtes,
**dadurch gekennzeichnet, dass** die LED's (25) auf ringförmigen, lichtdurchlässigen Trägern (10) vorgesehen sind, dass die Träger (10) zwischen innerem und äußerem Hüllrohr (1, 2) angeordnet sind und dass mehrere Träger (10) zwischen proximalem und distalem Ende des Schaftes im Ringraum zwischen innerem und äußerem Hüllrohr (1, 2) axial, kaskadenartig hintereinander angeordnet sind.

2. Endoskop nach Anspruch 1,
**dadurch gekennzeichnet, dass** die Träger (10) ringförmige Glasplättchen sind, auf welche die LED's (25) aufgebondet sind und Leiterbahnen (24) zur Stromversorgung der LED's (25) durch Bedampfen der Glasplättchen (10), vorzugsweise durch Goldbedampfung, angebracht sind.

3. Endoskop nach Anspruch 1 oder 2,
**dadurch gekennzeichnet, dass** der proximal letzte Träger proximalseitig eine Verspiegelung (11) aufweist.

4. Endoskop nach Anspruch 3,
**dadurch gekennzeichnet, dass** die Verspiegelung (11) aus einer auf den Träger (10) aufgedampften Metallschicht besteht.

5. Endoskop nach einem der Ansprüche 1 bis 4,
**dadurch gekennzeichnet, dass** die einander zugewandten Flächen des inneren und äußeren Hüllrohres (1, 2) ganz oder teilweise mit reflektierenden Schichten, vorzugsweise einer Hochglanzpolierung, ausgestattet sind.

6. Endoskop nach einem der Ansprüche 1 bis 5,
**dadurch gekennzeichnet, dass** am distalen Ende des Schaftes eine lichtdurchlässige Abschlussplatte (4) vorgesehen ist.

7. Endoskop nach einem der Ansprüche 1 bis 6,
**dadurch gekennzeichnet, dass** die Träger (10) für Padauslegung mit einer Metallegierung, vorzugsweise einer Nickel-Goldlegierung, beschichtet sind.

8. Endoskop nach Anspruch 7,
**dadurch gekennzeichnet, dass** auf die Träger (10) mit den Pads (24) ein die LED's enthaltender Wafer im Bondageverfahren aufgebracht ist.

9. Endoskop nach Anspruch 8,
**dadurch gekennzeichnet, dass** die LED's (25) mit Bubblen (15) abgedeckt sind.

10. Endoskop nach Anspruch 9,
**dadurch gekennzeichnet, dass** das Bubblematerial Epoxydharz, Urethaanacrylat oder Silikon ist, das vorzugsweise mit einem Fluoreszenzstoff versetzt ist.

11. Endoskop nach einem der Ansprüche 1 bis 10,
**dadurch gekennzeichnet, dass** die elektrischen Zuleitungen der LED's (25) im Ringraum zwischen innerem und äußerem Hüllrohr (1, 2) verlaufen und mit einem im Okular (8) angeordneten Steckkontakt (13) verbunden sind, welchem ein der Stromleitung dienender Stecker zugeordnet ist.

12. Endoskop nach einem der Ansprüche 1 bis 11,
**dadurch gekennzeichnet, dass** die elektrischen Zuleitungen der LED's im Ringraum zwischen innerem und äußerem Hüllrohr (1, 2) verlaufen und mit einer im Okular angeordneten Sekundär-Induktionsspule verbunden sind, welcher eine die Sekundär-Induktionsspule berührungslos umgebenden, ein magnetisches Feld erzeugenden Primärinduktionsspule zugeordnet ist.

13. Endoskop nach Anspruch 11 oder 12,
**dadurch gekennzeichnet, dass** die LED's (25) elektrisch parallelgeschaltet sind und dass die Stromversorgung über das innere und äußere Hüllrohr (1, 2) erfolgt, wobei die auf den Trägern (10) vorgesehenen Leiterbahnen, vorzugsweise die Padbeschichtungen (24), mit den Hüllrohren (1, 2) elektrisch leitend verbunden, vorzugsweise verlötet sind.

14. Endoskop nach einem der Ansprüche 1 bis 13, **gekennzeichnet durch** weiß scheinende oder monochromatisch leuchtende LED's (25).

15. Endoskop nach einem der Ansprüche 1 bis 14,
**dadurch gekennzeichnet, dass** dem Okularteller (8) ein Kamerakopf (28) zugeordnet ist, welcher über eine Steuereinheit (29) mit einem Monitor (30) verbunden ist.

16. Endoskop nach einem der Ansprüche 1 bis 15,
**dadurch gekennzeichnet, dass** in den Schaft eine opto-elektronische Bilderzeugungseinheit (17) integriert ist, welche über eine Steuereinheit mit einem Monitor verbunden ist.

## Claims

1. Endoscope comprising a shaft with coaxial outer and inner sheaths (1, 2), an optical system, preferably a rod lens system (6), arranged in the inner sheath (2) and a lens system (5) provided at the distal end or a collecting lens (16), as well as illumination units (10) arranged in the shaft and comprising light-emitting diodes (25) (LEDs) surrounding the inner sheath (2) in a ring-like manner for producing the light of illumination, **characterised in that** the LEDs (25) are provided on annular, transparent carriers (10), that the carriers (10) are arranged between the inner and outer sheaths (1, 2) and that a plurality of carriers (10) are arranged axially one behind the other in a cascade between the proximal and distal ends of the shaft in the annular space between the inner and outer sheaths (1, 2).

2. Endoscope according to claim 1, **characterised in that** the carriers 10 are annular glass plates to which the LEDs (25) are bonded and conductor tracks (24) for supplying current to the LEDs (25) are applied to the glass plates (10) by sputtering, preferably by gold sputtering.

3. Endoscope according to claim 1 or claim 2, **characterised in that** the proximally last carrier comprises a proximal mirror coating (11).

4. Endoscope according to claim 3, **characterised in that** the mirror coating (11) consists of a metal layer vapour-deposited on to the carrier (10).

5. Endoscope according to one of claims 1 to 4, **characterised in that** the surfaces of the inner and outer sheaths (1, 2) directed towards one another are provided completely or partially with reflecting layers, preferably with a mirror finish.

6. Endoscope according to one of claims 1 to 5, **characterised in that** a transparent end plate (4) is provided at the distal end of the shaft.

7. Endoscope according to one of claims 1 to 6, **characterised in that** the carriers (10) are coated with a metal alloy, preferably a nickel-gold alloy in order to provide pads.

8. Endoscope according to claim 7, **characterised in that** a wafer containing the LEDs is applied to the carriers (10) with the pads (24) by bonding.

9. Endoscope according to claim 8, **characterised in that** the LEDs (25) are covered in bubbles (15).

10. Endoscope according to claim 9, **characterised in that** the bubble material is epoxy resin, urethane acrylate or silicone, to which a fluorescent substance is preferably added.

11. Endoscope according to one of claims 1 to 10, **characterised in that** the electrical leads of the LEDs (25) extend in the annular space between the inner and outer sheaths (1, 2) and are connected to a plug contact (13) arranged in the eyepiece (8) with which a plug serving to conduct current is associated.

12. Endoscope according to one of claims 1 to 11, **characterised in that** the electrical leads of the LEDs extend in the annular space between the inner and outer sheaths 1, 2 and are connected to a secondary induction coil arranged in the eyepiece with which a primary induction coil generating a magnetic field and surrounding the secondary induction coil in a contactless manner is associated.

13. Endoscope according to claim 11 or claim 12, **characterised in that** the LEDs (25) are connected electrically in parallel and that the current is supplied via the inner and outer sheaths (1, 2), the conductor tracks, preferably the pad layers (24), provided on the carriers (10) being bonded, preferably soldered, to the sheaths (1, 2).

14. Endoscope according to one of claims 1 to 13, **characterised by** white or monochromatic LEDs (25).

15. Endoscope according to one of claims 1 to 14, **characterised in that** a camera head (28) is associated with the eyepiece disc (8) and is connected by means of a control unit (29) to a monitor (30).

16. Endoscope according to one of claims 1 to 15, **characterised in that** an optoelectronic image-forming unit (17) is integrated into the shaft and is connected by means of a control unit to a monitor.

## Revendications

1. Endoscope avec une tige dotée de gaines extérieure et intérieure coaxiales (1, 2), une optique disposée dans la gaine intérieure (2), de préférence une optique à lentille cylindriques (6), et un système lenticulaire (5) ou une lentille convergente (16) ainsi que des unités d'éclairage (10) disposées dans la tige dotée de diodes luminescentes (25) (LED) ceinturant la gaine intérieure (2) pour produire la lumière d'éclairage,
**caractérisé en ce que**
les LED (25) sont prévues sur des supports transparents annulaires (10), que les supports (10) sont disposés entre les gaines intérieure et extérieure (1, 2) et que plusieurs supports (10) sont disposés en cascade les uns derrière les autres entre les extrémités proximale et distale de la tige dans l'espace annulaire entre les gaines intérieure et extérieure (1, 2).

2. Endoscope selon la revendication 1,
**caractérisé en ce que**
les supports (10) sont des plaquettes de verre annulaires sur lesquelles les LED (25) sont liaisonnées et des pistes conductrices (24) pour l'alimentation électrique des LED (25) sont posées par vaporisation sur les plaquettes de verre (10), de préférence par vaporisation à l'or.

3. Endoscope selon la revendication 1 ou 2,
**caractérisé en ce que**
le dernier support proximal présente un dépôt réfléchissant (11) du côté proximal.

4. Endoscope selon la revendication 3,
**caractérisé en ce que**
le dépôt réfléchissant (11) est constitué d'une couche métallique vaporisée sur le support (10).

5. Endoscope selon une des revendications 1 à 4,
**caractérisé en ce que**
les faces orientées l'une vers l'autre des gaines intérieure et extérieure (1, 2) sont totalement ou partiellement dotées de couches réfléchissantes, de préférence d'un polissage à fort brillant.

6. Endoscope selon une des revendications 1 à 5,
**caractérisé en ce qu'**
à l'extrémité distale de la tige est prévue une plaque d'obturation transparente (4).

7. Endoscope selon une des revendications 1 à 6,
**caractérisé en ce que**
les supports (10) pour le tracé des plots sont recouverts d'un alliage métallique, de préférence un alliage nickel-or.

8. Endoscope selon la revendication 7,
**caractérisé en ce que**
sur les supports (10), avec les plots (24) on monte par liaisonnement une plaquette contenant les LED.

9. Endoscope selon la revendication 8,
**caractérisé en ce que**
les LED (25) sont recouvertes par des ampoules (15).

10. Endoscope selon la revendication 9,
**caractérisé en ce que**
le matériau des ampoules est de la résine époxy, de l'acrylate d'uréthane ou du silicone qui est de préférence mélangé avec une matière fluorescente.

11. Endoscope selon une des revendications 1 à 10,
**caractérisé en ce que**
les conducteurs électriques des LED (25) courent dans l'espace annulaire entre les gaines intérieure et extérieure (1, 2) et sont reliés avec un contact à fiche (13) disposé dans l'oculaire (8) auquel est associée une fiche servant pour l'alimentation électrique.

12. Endoscope selon une des revendications 1 à 11,
**caractérisé en ce que**
les conducteurs électriques des LED courent dans l'espace annulaire entre les gaines intérieure et extérieure (1, 2) et sont reliés avec une bobine d'induction secondaire disposée dans l'oculaire à laquelle est associée une bobine d'induction primaire entourant la bobine d'induction secondaire sans contact et générant un champ magnétique.

13. Endoscope selon la revendication 11 ou 12,
**caractérisé en ce que**
les LED (25) sont connectées électriquement en parallèle et que l'alimentation électrique se fait par les gaines intérieure et extérieure (1, 2), les pistes conductrices prévues sur les supports (10), de préférence des revêtements (24), étant reliées de manière électriquement conductrice, de préférence soudées, avec les gaines (1, 2).

14. Endoscope selon une des revendications 1 à 13,
**caractérisé par**
des LED (25) à lumière blanche ou monochromatique.

15. Endoscope selon une des revendications 1 à 14,
**caractérisé en ce qu'**
à l'oculaire (8) est associé une tête de caméra (28) qui est reliée par une unité de commande (29) avec un moniteur (30).

16. Endoscope selon une des revendications 1 à 15,
**caractérisé en ce que**
dans la tige est intégrée une unité d'imagerie optoélectronique (17) qui est reliée par une unité de commande avec un moniteur.
